# EUROPEAN PATENT SPECIFICATION

(11) **EP 4 023 752 B1**
(45) Date of publication and mention of the grant of the patent: **22.10.2025**
(21) Application number: 20306708.7
(22) Date of filing: 30.12.2020
(51) Int. Cl.: C12N 9/52, C07K 14/195

(54) **NOVEL PROTEASES AND USES THEREOF**
NEUARTIGE PROTEASEN UND VERWENDUNGEN DAVON
NOUVELLES PROTÉASES ET LEURS UTILISATIONS

(43) Date of publication of application: 06.07.2022
(73) Proprietor: Carbios, 63100 Clermont-Ferrand (FR)
(72) Inventor: BOUCHIBA, Younes, 63100 Clermont-Ferrand (FR); ANDRE, Isabelle, 63100 Clermont-Ferrand (FR); BARBRE, Sophie, 63100 Clermont-Ferrand (FR); DUQUESNE, Sophie, 63100 Clermont-Ferrand (FR)
(74) Representative: Cabinet Becker et Associés

(56) References cited:
- WO-A1-2019/122308
- WO-A1-2021/099337
- DATABASE EMBL [online] 2 September 2020 (2020-09-02), ANONYMOUS: "Actinomadura luteofluorescens subtilisin family serine protease", XP055811647, retrieved from EBI accession no. NYD47750 Database accession no. NYD47750

## Description

The present invention relates to novel proteases, more particularly to proteases having an improved thermostability compared to a parent protease and uses thereof for degrading polyester or polyester containing material, such as plastic products. The proteases of the invention are particularly suited to degrade polylactic acid and polylactic acid containing material.

### BACKGROUND

Proteases are able to catalyze the hydrolysis of a variety of polymers, including polyesters. In this context, proteases have shown promising effects in a number of industrial applications, including as detergents for dishwashing and laundry applications, as degrading enzymes for processing biomass and food, as biocatalysts in the detoxification of environmental pollutants or for the treatment of polyester fabrics in the textile industry. Likewise, the use of proteases as degrading enzymes for hydrolyzing polylactic acid (PLA) is of particular interest. Indeed, PLA is a bio-based polymer (i.e., a polymer derived from natural and/or renewable sources) that is used in a large number of technical fields, such as flexible and rigid packaging, bags, mulching films, as well as in the manufacture of clothes and carpets. Accordingly, PLA accumulation in landfills becomes an increasing ecological problem.

Among proteases, serine proteases (EC 3.4.21) are enzymes that cleave peptide amide bonds in proteins, in which serine serves as the nucleophilic amino acid in the enzyme active site. Serine proteases are found ubiquitously in both eukaryotes and prokaryotes. Numerous bacterial serine proteases have been identified initially in *Bacillus* and more recently in other mesophilic hosts. However, an increasing number of serine proteases have been isolated from thermophilic and hyperthermophilic bacteria.

Biological degradation, and more particularly enzymatic degradation, is considered as an interesting solution to decrease plastic waste accumulation. Indeed, enzymes are able to accelerate hydrolysis of polyester containing material, and more particularly of plastic products, even down to the monomer level. Furthermore, the hydrolysate (i.e., monomers and oligomers) can be recycled as material for the synthesis of new polymers. Recently, new plastic materials have been developed that integrate biological entities suitable for degrading at least one polymer of the plastic material, leading to the production of biodegradable plastic products. As an example, plastic products made of PLA and including proteases have been produced. Such biodegradable plastics may at least partially solve the problem of plastic build-up in landfill sites and natural habitats.

In this context, several proteases have been identified as candidate degrading enzymes. For instance, a protease of *Actinomadura sp.* (as described in WO 2016/062695) and variants thereof (as described in WO 2019/122308) exhibit capacity to degrade polyester, and more particularly polylactic acid.

However, there is still a need for proteases with improved thermostability. Proteases with increased thermostability would be easily processed in process of producing biodegradable plastics, and/or would allow enhancing efficiency of degrading and/or recycling processes of plastic products, and thereby enhancing the competitiveness of such processes.

### SUMMARY OF THE INVENTION

The present invention provides new variants of proteases exhibiting increased thermostability compared to their parent protease. These proteases are particularly useful in processes for degrading polyester(s) and/or plastic material and within plastic material and products containing polyester(s), such as polylactic acid (PLA). More particularly, the present invention provides variants of a protease having the amino acid sequence as set forth in SEQ ID N°1, referenced herein as the parent protease. The present invention further provides processes for degrading polyester(s) and/or plastic material and product containing polyester(s), and more particularly polylactic acid (PLA) and/or plastic material and product containing PLA, using a variant of the invention.

In this regard, it is an object of the invention to provide a protease variant which comprises an amino acid sequence which (i) has at least 90%, 95%, 96%, 97%, 98% or 99% identity to the full length amino acid sequence set forth in SEQ ID N°1, and (ii) has at least one combination of substitutions selected from the group consisting of N262C + T266C, A169C + T176C, W9C + S182C, N173C + F243C, A187C + S203C, C164A + A172C, S194E + H197D + S264P and N253C + N272C, or comprises at least the replacement of the portion of amino acid sequence corresponding to residues [L210 to G213] with the sequence YTSETA or YTSSTA, (iii) comprises the combination D40 + H71 + S221 as in the parent protease, wherein the positions are numbered by reference to the amino acid sequence set forth in SEQ ID N°1, and (iv) has a polyester degrading activity, and (v) exhibits increased thermostability compared to the protease of SEQ ID N°1.

Advantageously, the protease of the invention exhibits an increased thermostability compared to the thermostability of the protease of SEQ ID N°1 at temperatures between 40°C to 90°C, between 40°C and 80°C, between 40°C and 70°C, between 40°C and 60°C, between 40°C and 50°C, between 50 and 80°C, between 60°C and 80°C, particularly at 70°C +/- 5°C.

It is another object of the invention to provide a nucleic acid encoding a protease as defined above. The present invention also relates to an expression cassette or an expression vector comprising said nucleic acid, and to a host cell comprising said nucleic acid, expression cassette or vector.

It is a further object of the invention to provide a method of producing a protease as defined above comprising:
(a) culturing a host cell as defined above under conditions suitable to express a nucleic acid encoding a protease; and optionally
(b) recovering said protease from the cell culture.

The present invention also relates to a method of degrading a plastic product containing at least one polyester, preferably at least PLA, comprising
(a) contacting the plastic product with a protease or host cell as defined above, thereby degrading the plastic product; and optionally
(b) recovering monomers and/or oligomers, preferably monomers and/or oligomers of lactic acid (LA).

The present invention also relates to a polyester containing material comprising a protease or host cell according to the invention. The present invention relates more preferably to a polylactic acid (PLA) containing material comprising a protease or host cell or composition according to the invention. The invention also provides a process for producing such polyester containing material comprising a step of mixing a polyester, preferably PLA, and a protease or host cell or composition according to the invention, wherein the mixing step is performed at a temperature at which the polyester is in a partially or totally molten state, preferably during an extrusion process.

The present invention further relates to the use of a protease as described above for degrading a polyester containing material, more preferably a PLA containing material.

### DETAILED DESCRIPTION OF THE INVENTION

### Definitions

The present disclosure will be best understood by reference to the following definitions.

Herein, the terms *"peptide", "polypeptide", "protein", "enzyme"* refer to a chain of amino acids linked by peptide bonds, regardless of the number of amino acids forming said chain. The amino acids are herein represented by their one-letter or three-letters code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp ) and Y: tyrosine (Tyr).

The term *"protease"* or *"peptidase"* refers to an enzyme which belongs to a class of hydrolases classified as EC 3.4 according to Enzyme Nomenclature that is able to catalyze the hydrolysis of a peptide bond in a peptide or a protein. The term *"serine protease"* refers to proteases classified as EC 3.4.21 according to the nomenclature of the Enzyme Commission.

The term *"parent protein"* refers to the protease having the amino acid sequence as set forth in SEQ ID N°1.

The terms *"mutant"* and *"variant"* may be used interchangeably to refer to polypeptides derived from a parent polypeptide and comprising at least one modification or alteration, i.e., a substitution, insertion, and/or deletion, at one or more positions. Variants may be obtained by various techniques well known in the art. In particular, examples of techniques for altering a DNA sequence encoding the parent protein, include, but are not limited to, site-directed mutagenesis, random mutagenesis and synthetic oligonucleotide construction. Thus, the terms *"modification"* and *"alteration"* as used herein, in relation to a particular position, means that at least the amino acid in this particular position has been modified compared to the amino acid in this particular position in the parent protein.

*A "substitution"* means that an amino acid residue is replaced by another amino acid residue. Preferably, the term "substitution" refers to the replacement of an amino acid residue by another selected from the naturally-occurring standard 20 amino acid residues, rare naturally occurring amino acid residues (e.g. hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine, aminobutyric acid, ornithine, norleucine, norvaline), and non-naturally occurring amino acid residue, often made synthetically, (e.g. cyclohexyl-alanine). Preferably, the term *"substitution"* refers to the replacement of an amino acid residue by another selected from the naturally-occurring standard 20 amino acid residues (G, P, A, V, L, I, M, C, F, Y, W, H, K, R, Q, N, E, D, S and T). The sign "+" indicates a combination of substitutions. In the present document, the following terminology is used to designate a substitution: Y167R denotes that amino acid residue Tyrosine (Y) at position 167 of a parent sequence is substituted by an Arginine (R). Y167V/I/M denotes that amino acid residue Tyrosine (Y) at position 167 of a parent sequence is substituted by one of the following amino acids: Valine (V), Isoleucine (I), or Methionine (M). The substitution can be a conservative or non-conservative substitution. Examples of conservative substitutions are within the groups of basic amino acids (arginine, lysine and histidine), acidic amino acids (glutamic acid and aspartic acid), polar amino acids (glutamine, asparagine and threonine), hydrophobic amino acids (methionine, leucine, isoleucine, cysteine and valine), aromatic amino acids (phenylalanine, tryptophan and tyrosine), and small amino acids (glycine, alanine and serine).

The term *"deletion",* used in relation to an amino acid, means that the amino acid has been removed or is absent.

The term *"insertion",* used in relation to a portion of amino acid sequence means that one or more amino acids have been added within the targeted portion of amino acid sequence. Thus, the terminology "insertion of at least one amino acid in the portion of amino acid sequence corresponding to residues [A208 to N215]" means that at least one amino acid, i.e., one or more amino acids, are added between adjacent amino acid residues from A208 to N215. When two or more amino acid residues are inserted within a targeted portion of amino acid sequence, said residues may be either inserted between same amino acids or between different amino acids of the targeted portion of amino acids.

Unless otherwise specified, the positions disclosed in the present application are numbered by reference to the amino acid sequence set forth in SEQ ID N°1. For ease of reference, unless otherwise specified, the numbering of amino acid residues after an insertion is not changed, i.e., the amino acid residues inserted in the protease variant are not taken into consideration in the numbering .

As used herein, the term *"sequence identity"* or *"identity"* refers to the number (or fraction expressed as a percentage %) of matches (identical amino acid residues) between two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithm (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. For purposes herein, % amino acid sequence identity values refers to values generated using the pair wise sequence alignment program EMBOSS Needle that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5. The percentage of identity is calculated by determining the number of identical positions between these two sequences, by dividing this number by the total number of compared positions, and by multiplying the result obtained by 100 to get the percentage of identity between these two sequences.

The term *"recombinant"* refers to a nucleic acid construct, a vector, a polypeptide or a cell produced by genetic engineering.

The term *"expression",* as used herein, refers to any step involved in the production of a polypeptide such as transcription, post-transcriptional modification, translation, post-translational modification, or secretion.

According to the invention, *"oligomers"* refer to molecules containing from 2 to about 20 monomers.

In the present description, *"polyesters"* encompass polylactic acid (PLA), polyethylene terephthalate (PET), polytrimethylene terephthalate (PTT), polybutylene terephthalate (PBT), polyethylene isosorbide terephthalate (PEIT), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), or poly(ethylene adipate) (PEA), as well as any blends/mixtures of these polymers. In a particular embodiment, "polyester" also encompasses poly(glycolic acid) (PGA) or poly(lactic-co-glycolic acid) (PLGA) as well as any blends/mixtures of these polymers.

In the context of the invention, a *"polyester containing material"* or *"polyester containing product"* refers to a product, such as plastic product, comprising at least one polyester in crystalline, semi-crystalline or totally amorphous form. In a particular embodiment, the polyester containing material refers to any item made from at least one plastic material, such as plastic sheet, tube, rod, profile, shape, film, massive block, fiber, textiles, etc., which contains at least one polyester, and possibly other substances or additives, such as plasticizers, mineral or organic fillers. In another particular embodiment, the polyester containing material refers to textile, fabrics or fibers comprising at least one polyester. In another particular embodiment, the polyester containing material refers to plastic waste or fiber waste comprising at least one polyester. In another particular embodiment, the polyester containing material refers to a plastic compound, or plastic formulation, in a molten or solid state, suitable for making a plastic product.

Within the context of the invention, the term *"improved thermostability"* indicates an increased ability of an enzyme to resist to changes in its chemical and/or physical structure at high temperatures, and more particularly at temperatures between 40°C and 90°C, such as 70°C +/-5°C, as compared to the protease of SEQ ID N°1. Such an increase is typically of about 1-fold, 2-fold, 3-fold, 4-fold, 5-fold, or more. In particular, the proteases of the present invention can have an increased residual degrading activity at a temperature between 40°C and 90°C, such as 70°C, as compared to the protease of SEQ ID N°1. Alternatively or in addition, the proteases of the present invention can have an increased half-life at a temperature between 40°C and 90°C, such as 70°C, as compared to the protease of SEQ ID N°1.

Advantageously, the variant of the invention shows an improved thermostability during an extrusion process, and more particularly during an extrusion process implemented at a temperature comprised between 50°C and 250°C, preferably between 130°C and 180°C.

The thermostability of a protein may be evaluated by the one skilled in the art, according to methods known per se in the art. For instance, thermostability can be assessed by measuring the residual protease activity and/or the residual polyester depolymerization activity (i.e., polyester degrading activity) of the enzyme after incubation at different temperatures and comparing with the residual protease activity and/or residual polyester depolymerization activity of the parent protease. The ability to perform multiple rounds of polyester's depolymerization assays at different temperatures can also be evaluated. A rapid and qualitative test may consist on the evaluation, by halo diameter measurement, of the enzyme ability to degrade a solid polyester compound dispersed in an agar plate after incubation at different temperatures. Alternatively or in addition, a Differential Scanning Fluorimetry (DSF) or Nano differential scanning fluorimetry (Nano-DSF) may be performed to assess the thermostability of a protein/enzyme by measuring the melting temperature (Tm) of said protease. Alternatively, the Tm can be assessed by analysis of the protein folding using circular dichroism. Both methods are used to quantify the change in thermal denaturation temperature of a protein and thereby to determine its melting temperature (Tm). In the context of the invention the *"melting temperature (Tm)"* of a given protein corresponds to the temperature at which half of protein population considered is denatured (unfolded or misfolded). The Tm may be measured using circular dichroism as exposed in the experimental part. In the context of the invention, comparisons of Tm are performed with Tm that are measured under same conditions (e.g. pH, nature and amount of polyesters, etc.). Advantageously, the protease of the invention exhibits an increased melting temperature (Tm) of about 1°C, 2°C, 3°C, 4°C, 5°C, 10°C or more, as compared to the protease of SEQ ID N°1. In particular, proteases of the present invention can have an increased half-life at a temperature between 40°C and 80°C, as compared to the protease of SEQ ID N°1.

### Novel proteases

By working on development of novel proteases having improved thermostability as compared to enzymes currently available, the inventors have further worked on the serine protease having the amino acid sequence of SEQ ID N°1 and have developed variants thereof that exhibit improved thermostability. The novel proteases derived from SEQ ID N°1 are advantageously particularly suited to degrade polyester or polyester-containing products. Interestingly, these new proteases have superior properties for use in industrial processes. The proteases newly developed exhibit an improved thermostability, particularly under industrial production conditions of degradable plastic products and/or under environmental degradation conditions of plastic products. Interestingly, the degrading activity of such new proteases is not impaired or not significatively impaired as compared to the degrading activity of the parent protease.

SEQ ID N°1:








It is an object of the present invention to provide a protease which comprises an amino acid sequence which (i) has at least 90%, 95%, 96%, 97%, 98% or 99% identity to the full length amino acid sequence set forth in SEQ ID N°1, and (ii) has at least one combination of substitutions selected from the group consisting of N262C + T266C, A169C + T176C, W9C + S182C, N173C + F243C, A187C + S203C, C164A + A172C, S194E + H197D + S264P and N253C + N272C, or comprises at least the replacement of the portion of amino acid sequence corresponding to residues [L210 to G213] with the sequence YTSETA or YTSSTA , (iii) comprises the combinations D40 + H71 + S221 as in the parent protease, wherein the positions are numbered by reference to the amino acid sequence set forth in SEQ ID N°1, and (iv) has a polyester degrading activity, and (v) exhibits increased thermostability compared to the protease of SEQ ID N°1.

In the context of the present invention, the term "replacement" is used to designate the modification of a target portion of amino acid sequence either by use of insertion(s) and/or substitution(s) or by replacement of the whole target portion of amino acid sequence with another.

A protease is disclosed, which comprises at least one insertion of at least one amino acid residue in the portion of amino acid sequence corresponding to residues [W209 to T214], preferably in the portion corresponding to residues [L210 to G213] and at least one substitution, preferably at least two, three or four substitutions selected from L210S/T/Y, N211E/D/S/T, G212E/D/S/T and G213A/E/D/S/T. Alternatively, said protease may be obtained by replacement of the portion of amino acid sequence corresponding to residues [W209 to T214], preferably of the portion corresponding to residues [L210 to G213] with the corresponding portion of amino acid sequence.

The protease may comprise at least one insertion of two amino acids and four substitutions in the portion of amino acid sequence corresponding to [L210 to G213]. More preferably, the protease comprises an insertion of two amino acids between position G212 and G213, preferably selected from E/D/S/T + T/S and the combination of four substitutions selected from L210Y+ N211T/S + G212S/T + G213A, so that the portion of amino acid sequence corresponding to residues [L210 to G213] is replaced with the amino acid sequence consisting of Y-T/S-S/T-E/D/S/T-T/S-A, preferably with the amino acid sequence selected from Y-T-S-E-T-A or Y-T-S-S-T-A. Alternatively, said protease may be obtained by replacement of the portion of amino acid sequence corresponding to residues [L210 to G213] with the portion of amino acid sequence consisting of Y-T/S-S/T-E/D/S/T-T/S-A, preferably with the portion of amino acid sequence selected from Y-T-S-E-T-A or Y-T-S-S-T-A.

The protease may comprise at least one insertion of at least one amino acid residue in the portion of amino acid sequence corresponding to residues [L210 to G213], and at least one substitution, preferably at least two, three or four substitutions selected from L210S/T/Y, N211E/D/S/T, G212E/D/S/T and G213A/E/D/S/T, preferably a combination of substitution selected from L210S/T/Y + N211E/S/T + G212E/S/T + G213A/E/S/T, so that the portion of amino acid sequence corresponding to residues [L210 to G213] is replaced with the amino acid sequence selected from Y-T-S-E-T-A or Y-T-S-S-T-A.

In addition to any of the above embodiments, the protease may further comprise at least one amino acid substitution at a position selected from G31, V34, N35, Y44, A87, V90, A117, N143, A153, A169, A172, A174, T175, T176, A179, R186, A187, H197, S203, N211, T230 and R247, preferably G31C, V34C, N35C, Y44A, A87C, V90C, A117C, N143D, A153C, A169C, A172C, A174C, T175C, T176C, A179C, R186C, A187C, H197D/E, S203C, N211S, T230A/V and R247C, more preferably selected from A169C, A172C, T176C, A187C, H197D/E, S203C and N211S. Preferably, said protease further comprises at least one amino acid substitution at position H197, more preferably selected from H197D/E, even more preferably H197D.

In a particular embodiment, the protease has at least 90%, 95%, 96%, 97%, 98% or 99% identity to the full length amino acid sequence set forth in SEQ ID N°1 and comprises the replacement of the portion of amino acid sequence corresponding to residues [L210 to G213] with a portion of amino acid sequence consisting of Y-T-S-E-T-A or Y-T-S-S-T-A, and at least one substitution or at least one combination of substitutions selected from T17E, S194E + H197D, T17E + S194E + H197D.

In another embodiment, the protease comprises at least one combination of substitutions selected from the group consisting in N262C + T266C, A169C + T176C, W9C + S182C/, N173C + F243C, A187C + S203C, C164A + A172CS194E + H197D + S264P and N253C + N272C.

In another embodiment, the protease comprises at least the replacement of the portion of amino acid sequence [L210 to G213] with the sequence YTSETA or YTSSTA, and optionally further comprises at least a substitution or combination of substitutions selected from T17E, S194E + H197D, and T17E + S194E + H197D.

In an embodiment, the protease comprises at least one amino acid residue selected from C68, C100, C164, C195, V75, L98, F101, G102, L103, G104, T105, L106, V109, N127, L130, G131, G132, G133, S135, L138, A155, G157, E159, R166, I217, S218, M222, A223, T224, P225 as in the parent protease, i.e., the protease of the invention is not modified at one, two, three, etc., or all of these positions.

Any protease of the invention comprises the amino acid residues D40, H71 and S221 forming the catalytic site, as in the parent protease, i.e. the protease of the invention is not modified at all of these positions. The protease comprises the combination D40 + H71 + S221, as in the parent protease.

In addition, any protease of the invention may comprise at least one amino acid residue selected from C68, C100, C164 or C195 forming disulfide bonds, as in the parent protease, i.e. the protease of the invention is not modified at one, two or more of these positions. Preferably, the protease comprises the combination C68 + C100, C164 + C195 or C68 + C100 + C164 + C195 as in the parent protease.

In an embodiment, any protease of the invention comprises at least one combination selected from D40 + H71 + S221 + C68 + C100, D40 + H71 + S221 + C164 + C195 or D40 + H71 + S221 + C68 + C100 + C164 + C195 as in the parent protease.

Alternatively or in addition, any protease of the invention comprises at least one amino acid residue selected from V75, L98, F101, G102, L103, G104, T105, L106, V109, N127, L130, G131, G132, G133, S135, L138, A155, G157, E159, R166, I217, S218, M222, A223, T224, or P225 which correspond to residues in contact with the polyester (e.g., PLA) during the degradation reaction, as in the parent protease, i.e. the protease of the invention is not modified at one, two or more of these positions.

Preferably, the protease comprises at least one amino acid residue selected from F101, L103 or L106 as in the parent protease which are positions known to be important for improved degrading activity. More preferably, the protease comprises the combination F101 + L103 + L106 as in the parent protease. Alternatively, the protease comprises the combination F101 + L103 as in the parent protease and at least the substitution L106I. Alternatively or in addition, the protease of the invention comprises at least one amino acid substitution selected from F101S/L/M/W/Y, L103S, L106I/T, G131I, or G133K, wherein the positions are numbered by reference to the amino acid sequence set forth in SEQ ID N°1.

### Activity

Advantageously, the degrading activity of the variant proteases of the invention is not impaired (i.e., not decreased) or not significatively impaired compared to the degrading activity of the parent protease of SEQ ID N°1. More advantageously, the polymer degrading activity of the variants is improved compared to the degrading activity of the parent protease. Within the context of the invention, the term *"degrading activity"* indicates an ability of the enzyme to degrade a polyester, preferably PLA or a plastic product comprising at least a polyester, preferably at least PLA.

The activity of a protein may be evaluated by the one skilled in the art, according to methods known per se in the art. For instance, the activity can be assessed by the measurement of the specific protease activity rate, the measurement of the hydrolysis of pNA (N-succinyl-Ala-Ala-Ala-p-nitroanilide), the measurement of the specific polyester's depolymerization activity rate, the measurement of the rate to degrade a solid polyester compound or protein dispersed in an agar plate, the measurement of the decrease of the turbidity of an emulsion containing a polyester, or the measurement of the specific polyester's depolymerization activity rate in reactor.

Within the context of the invention, the term *"specific degrading activity"* for a targeted polyester designates the initial rate of monomers and/or oligomers, in mg, released per hour and per mg of enzyme under suitable conditions of temperature, pH and buffer, when contacting a plastic product containing said targeted polyester with a protease according to the invention. As an example, the specific degrading activity for PLA corresponds to the mg of lactic acid and dimers of lactic acid produced per hour and per mg of enzyme, or to the µmol of PLA hydrolyzed/min and per mg of enzyme, as determined in the linear part of the hydrolysis curve.

Advantageously, the protease of the invention has a polyester degrading activity corresponding to at least 50% of the polyester degrading activity rate of the protease of SEQ ID N°1, preferably at least 70%, more preferably at least 80%, even more preferably at least 90%, particularly 100%. In another embodiment, the protease of the invention has a polyester degrading activity corresponding to at least 100% of the polyester degrading activity rate of the protease of SEQ ID N°1, preferably at least 110%, more preferably at least 120%, even more preferably at least 130%, 140%, 150%, 160%, 170%, 180%, 190%, 200%.

In a particular embodiment, the protease comprises the replacement of the portion of amino acid sequence [L210 to G213] with the sequence YTSETA or with the sequence YTSSTA, and optionally at least one substitution or combination of substitutions selected from T17E or S194E + H197D, and its degrading activity is not impaired as compared to SEQ ID N°1.

In a particular embodiment, the protease comprises the replacement of the portion of amino acid sequence [L210 to G213] with the sequence YTSETA and exhibits an increased degrading activity.

### Propeptide

Advantageously, the protease variant comprises, at the N-terminal end of the amino acid sequence having % identity with SEQ ID N°1, an amino acid sequence acting as a *"propeptide"* which is involved in the 3D folding and the maturation of the protease.

Particularly, the protease variant comprises a propeptide sequence, which has at least 75%, 80%, 85%, 90%, 95%, 96%, 97%, 98%, 99% or 100% identity to the full length amino acid sequence set forth in SEQ ID N°2.

SEQ ID N°2:

APAVPVAMAAAGQGVAGQYIVTLKKGVSVDSTVAKRGIRTQHRFGKVLNGFSAKLT
DDQLSKLRTTPGVASIEQDAVITVD

According to a particular embodiment, the protease variant comprises at the N-terminal end an amino acid sequence which has at least 75%, 80%, 85%, 90%, 95%, 96%, 97%, 98% or 99% identity to the full length amino acid sequence set forth in SEQ ID N°2, and has at least one amino acid substitution at a position selected from M8, D75, A76, I78 or D81 wherein the positions are numbered by reference to the amino acid sequence set forth in SEQ ID N°2.

According to the invention, the targeted amino acid(s) may be replaced by any one of the other amino acids selected from standard naturally-occurring amino acid residues, rare naturally occurring amino acid residues and non-naturally occurring amino acid residue. Preferably, the targeted amino acid(s) may be replaced by any one of the 19 other amino acids.

### Polyester degrading activity

It is an object of the invention to provide new enzymes having a polyester degrading activity, preferably a polylactic acid degrading activity. Preferably, the protease of the invention exhibits an increased polyester degrading activity compared to the protease of SEQ ID N°1.

Advantageously, the protease variant of the invention exhibits a polyester degrading activity at least in a range of temperatures from 10°C to 90°C, preferably from 20°C to 70°C, more preferably from 30°C to 60°C, even more preferably at 45°C +/-5°C.

In another embodiment, the protease variant exhibits a polyester degrading activity at 70°C +/-5°C. In a particular embodiment, a polyester degrading activity is still measurable at a temperature between 40°C and 60°C, preferably between 50°C and 60°C. In another particular embodiment, the polyester degrading activity is still measurable at a temperature between 10°C and 30°C, preferably between 15°C and 28°C, corresponding to the mean temperature in the natural environment.

In a particular embodiment, the protease variant of the invention has an increased polyester degrading activity at a given temperature, compared to the protease of SEQ ID N°1, and more particularly at a temperature between 20°C and 80°C, more preferably between 30°C and 70°C, even more preferably between 40°C and 60°C, even more preferably at 45°C+/-5°C. In another embodiment, the protease variant exhibits an increased polyester degrading activity at 70°C +/-5°C.

In a particular embodiment, the protease variant has a polyester degrading activity at 45°C at least 5% higher than the polyester degrading activity of the protease of SEQ ID N°1, preferably at least 10%, 20%, 50%, 100%, 200%, 300%, 500% or higher.

In a particular embodiment, the protease variant of the invention has an increased polyester degrading activity, compared to the protease of SEQ ID N°1, at a temperature between 10°C and 30°C, more preferably between 15°C and 30°C, even more preferably between 20°C and 30°C, even more preferably at 28°C +/-2°C. In a particular embodiment, the protease variant has a polyester degrading activity at 28°C +/-2°C at least 5% higher than the polyester degrading activity of the protease of SEQ ID N°1, preferably at least 10%, 20%, 50%, 100%, 200%, 300%, 500% or higher.

In a particular embodiment, the protease variant of the invention exhibits a measurable polyester degrading activity at least in a range of pH from 5 to 11, preferably in a range of pH from 7 to 10, more preferably in a range of pH from 7.5 to 9, even more preferably at both pH 7.5 and 9.

### Nucleic acids, expression cassette, vector, host cell

It is a further object of the invention to provide a nucleic acid encoding a protease as defined above.

As used herein, the term *"nucleic acid", "nucleic sequence," "polynucleotide", "oligonucleotide"* and *"nucleotide sequence"* are used interchangeably and refer to a sequence of deoxyribonucleotides and/or ribonucleotides. The nucleic acids can be DNA (cDNA or gDNA), RNA, or a mixture thereof. It can be in single stranded form or in duplex form or a mixture thereof. It can be of recombinant, artificial and/or synthetic origin and it can comprise modified nucleotides, comprising for example a modified bond, a modified purine or pyrimidine base, or a modified sugar. The nucleic acids of the invention can be in isolated or purified form, and made, isolated and/or manipulated by techniques known per se in the art, e.g., cloning and expression of cDNA libraries, amplification, enzymatic synthesis or recombinant technology. The nucleic acids can also be synthesized in vitro by well-known chemical synthesis techniques, as described in, e.g., Belousov (1997) Nucleic Acids Res. 25:3440-3444.

The invention also encompasses nucleic acids which hybridize, under stringent conditions, to a nucleic acid encoding a protease as defined above. Preferably, such stringent conditions include incubations of hybridization filters at about 42°C for about 2.5 hours in 2 X SSC/0.1%SDS, followed by washing of the filters four times of 15 minutes in 1 X SSC/0.1% SDS at 65° C. Protocols used are described in such reference as Sambrook et al. (Molecular Cloning: a Laboratory Manual, Cold Spring Harbor Press, Cold Spring Harbor N.Y. (1988)) and Ausubel (Current Protocols in Molecular Biology (1989)).

The invention also encompasses nucleic acids encoding a protease of the invention, wherein the sequence of said nucleic acids, or a portion of said sequence at least, has been engineered using optimized codon usage.

Alternatively, the nucleic acids according to the invention may be deduced from the sequence of the protease according to the invention and codon usage may be adapted according to the host cell in which the nucleic acids shall be transcribed. These steps may be carried out according to methods well known to one skilled in the art and some of which are described in the reference manual Sambrook et al. (Sambrook et al., 2001).

Nucleic acids of the invention may further comprise additional nucleotide sequences, such as regulatory regions, i.e., promoters, enhancers, silencers, terminators, signal peptides and the like that can be used to cause or regulate expression of the polypeptide in a selected host cell or system. Alternatively, or in addition, nucleic acids of the invention may further comprise additional nucleotide sequences encoding fusion proteins, such as maltose binding protein (MBP) or glutathion S transferase (GST) that can be used to favor polypeptide expression and/or solubility.

The present invention further relates to an expression cassette comprising a nucleic acid according to the invention operably linked to one or more control sequences that direct the expression of said nucleic acid in a suitable host cell. The term *"expression cassette"* denotes a nucleic acid construct comprising a coding region, i.e. a nucleic acid of the invention, and a regulatory region, i.e. comprising one or more control sequences (e.g., transcriptional promoter and/or transcription terminator). The control sequence may include a promoter that is recognized by a host cell or an *in vitro* expression system for expression of a nucleic acid encoding a protease of the present invention. The promoter contains transcriptional control sequences that mediate the expression of the enzyme. The promoter may be any polynucleotide that shows transcriptional thermostability in the host cell including mutant, truncated, and hybrid promoters, and may be obtained from genes encoding extracellular or intracellular polypeptides either homologous or heterologous to the host cell. The control sequence may also be a transcription terminator, which is recognized by a host cell to terminate transcription. The terminator is operably linked to the 3'-terminus of the nucleic acid encoding the protease. Any terminator that is functional in the host cell may be used in the present invention. Typically, the expression cassette comprises, or consists of, a nucleic acid according to the invention operably linked to a transcriptional promoter and a transcription terminator.

The invention also relates to a vector comprising a nucleic acid or an expression cassette as defined above.

The term *"vector"* refers to DNA or RNA molecule used as a vehicle to transfer recombinant genetic material into a host cell. The vector may be linear or circular, single- or double-stranded DNA or RNA. The vector may be integrative or extrachromosomal, or autoreplicative. Preferably, the expression vector is a linear or circular double stranded DNA molecule. The major types of vectors are plasmids, bacteriophages, viruses, fosmids, cosmids, and artificial chromosomes. The vector itself is generally a DNA or RNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences encoding a polypeptide. As used herein, the term *"expression vector"* means a DNA or RNA molecule that comprises an expression cassette of the invention. Generally, the regulatory elements that are present in an expression vector include a transcriptional promoter, a ribosome binding site, a terminator, and optionally present operator. Preferably, an expression vector also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses. Expression vectors providing suitable levels of polypeptide expression in different hosts are well known in the art. The choice of the vector will typically depend on the compatibility of the vector with the host cell into which the vector is to be introduced. Preferably, the expression vector is a linear or circular double stranded DNA molecule.

It is another object of the invention to provide a host cell comprising a nucleic acid, an expression cassette or a vector as described above. The present invention thus relates to the use of a nucleic acid, expression cassette or vector according to the invention to transform, transfect or transduce a host cell. The choice of the vector will typically depend on the compatibility of the vector with the host cell into which it must be introduced.

According to the invention, the host cell may be transformed, transfected or transduced in a transient or stable manner. The expression cassette or vector of the invention is introduced into a host cell so that the cassette or vector is maintained as a chromosomal integrant or as a self-replicating extra-chromosomal vector. The term "host cell" also encompasses any progeny of a parent host cell that is not identical to the parent host cell due to mutations that occur during replication. The host cell may be any cell useful in the production of a variant of the present invention, e.g., a prokaryote or a eukaryote. The prokaryotic host cell may be any Gram-positive or Gram-negative bacterium. The host cell may also be a eukaryotic cell, such as a yeast, fungal, mammalian, insect or plant cell. In a particular embodiment, the host cell is selected from the group of *Escherichia coli, Bacillus, Streptomyces, Trichoderma, Aspergillus, Saccharomyces, Pichia, Thermus, Actinomadura* or *Yarrowia.* More preferably, the host cell is selected from the group of *Escherichia coli, Bacillus, Aspergillus, and Trichoderma.*

The nucleic acid, expression cassette or expression vector according to the invention may be introduced into the host cell by any method known by the skilled person, such as electroporation, conjugation, transduction, competent cell transformation, protoplast transformation, protoplast fusion, biolistic "gene gun" transformation, PEG-mediated transformation, lipid-assisted transformation or transfection, chemically mediated transfection, lithium acetate-mediated transformation, liposome-mediated transformation.

Optionally, more than one copy of a nucleic acid, cassette or vector of the present invention may be inserted into a host cell to increase production of the variant.

In a particular embodiment, the host cell is a recombinant microorganism. The invention indeed allows the engineering of microorganisms with improved capacity to degrade polyester containing material. For instance, the sequence of the invention may be used to complement a wild type strain of a fungus or bacterium already known as able to degrade polyester, in order to improve and/or increase the strain capacity.

### Production of protease variants

It is another object of the invention to provide a method of producing a protease variant of the invention, comprising expressing a nucleic acid encoding the protease and optionally recovering the protease.

In particular, the present invention relates to *in vitro* methods of producing a protease of the present invention comprising (a) contacting a nucleic acid, cassette or vector of the invention with an *in vitro* expression system; and (b) recovering the protease produced. *In vitro* expression systems are well-known by the person skilled in the art and are commercially available.

Preferably, the method of production comprises
(a) culturing a host cell that comprises a nucleic acid encoding a protease of the invention under conditions suitable to express the nucleic acid; and optionally
(b) recovering said protease from the cell culture.

Advantageously, the host cell is a recombinant *Bacillus,* recombinant *E. coli,* recombinant *Aspergillus,* recombinant *Trichoderma,* recombinant *Streptomyces,* recombinant *Saccharomyces,* recombinant *Pichia,* recombinant *Thermus,* recombinant *Actinomadura* or recombinant *Yarrowia.* Preferably, the host cell is selected from recombinant *Bacillus,* recombinant *E. coli,* recombinant *Aspergillus,* recombinant *Trichoderma.*

The host cells are cultivated in a nutrient medium suitable for production of polypeptides, using methods known in the art. For example, the cell may be cultivated by shake flask cultivation, or small-scale or large-scale fermentation (including continuous, batch, fed- batch, or solid state fermentations) in laboratory or industrial fermentors performed in a suitable medium and under conditions allowing the enzyme to be expressed and/or isolated. The cultivation takes place in a suitable nutrient medium, from commercial suppliers or prepared according to published compositions (e.g., in catalogues of the American Type Culture Collection) or any other culture medium suitable for cell growth.

If the protease is excreted into the nutrient medium, the protease can be recovered directly from the culture supernatant. Conversely, the protease can be recovered from cell lysates or after permeabilization. The protease may be recovered using any method known in the art. For example, the protease may be recovered from the nutrient medium by conventional procedures including, but not limited to, collection, centrifugation, filtration, extraction, spray-drying, evaporation, or precipitation. Optionally, the protease may be partially or totally purified by a variety of procedures known in the art including, but not limited to, thermal chock, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction to obtain substantially pure polypeptides.

The protease may be used as such, in purified form, either alone or in combination with additional enzymes, to catalyze enzymatic reactions involved in the degradation and/or recycling of polyester(s) and/or polyester containing materials, such as plastic products containing polyester. The protease may be in soluble form, or on solid phase. In particular, it may be bound to cell membranes or lipid vesicles, or to synthetic supports such as glass, plastic, polymers, filter, membranes, e.g., in the form of beads, columns, plates and the like.

### Composition

It is a further object of the invention to provide a composition comprising a protease or a host cell of the invention or extract thereof. In the context of the invention, the term *"composition"* encompasses any kind of compositions comprising a protease or host cell of the invention. In a particular embodiment, the protease is in isolated or at least partially purified form.

The composition may be liquid or dry, for instance in the form of a powder. In some embodiments, the composition is a lyophilisate. For instance, the composition may comprise the protease and/or host cells encoding the protease of the invention or extract thereof containing said protease, and optionally additives such as excipients and/or reagents etc. Appropriate excipients encompass buffers commonly used in biochemistry, agents for adjusting pH, preservatives such as sodium benzoate, sodium sorbate or sodium ascorbate, conservatives, protective or stabilizing agents such as starch, dextrin, arabic gum, salts, sugars e.g. sorbitol, trehalose or lactose, glycerol, polyethyleneglycol, polyethene glycol, polypropylene glycol, propylene glycol, divalent ions such as calcium, sequestering agent such as EDTA, reducing agents, amino acids, a carrier such as a solvent or an aqueous solution, and the like. The composition of the invention may be obtained by mixing the protease with one or several excipients.

The composition of the invention may comprise from 0.1% to 99.9%, preferably from 0.1% to 50%, more preferably from 0.1% to 30%, even more preferably from 0.1% to 5% by weight of the protease of the invention and from 0.1% to 99.9%, preferably from 50% to 99.9%, more preferably from 70% to 99.9%, even more preferably from 95% to 99.9% by weight of excipient(s). A preferred composition comprises between 0.1 and 5% by weight of the protease of the invention. In an embodiment, the composition of the invention may comprise from 0.1% to 40%, more preferably from 1% to 30%, even more preferably from 5% to 25% by weight of the protease of the invention and from 60% to 99.9%, preferably from 70% to 99%, more preferably from 75% to 95% by weight of excipient(s).

In a particular embodiment, the composition may further comprise additional polypeptide(s) exhibiting an enzymatic thermostability. The amounts of protease of the invention will be easily adapted by those skilled in the art depending e.g., on the nature of the polyester and/or polyester containing material to degrade and/or the additional enzymes/polypeptides contained in the composition.

In a particular embodiment, the protease of the invention is solubilized in an aqueous medium together with one or several additives such as excipients, especially excipients which are able to stabilize or protect the polypeptide from degradation. For instance, the protease of the invention may be solubilized in water, eventually with additional components, such as glycerol, sorbitol, dextrin, starch, glycol such as propanediol, salt, etc. The resulting mixture may then be dried so as to obtain a powder. Methods for drying such mixture are well known to the one skilled in the art and include, without limitation, lyophilisation, freeze-drying, spray-drying, supercritical drying, down-draught evaporation, thin-layer evaporation, centrifugal evaporation, conveyer drying, fluidized bed drying, drum drying or any combination thereof.

In a further particular embodiment, the composition of the invention comprises at least one host cell expressing a protease of the invention, or an extract thereof. An *"extract of a cell"* designates any fraction obtained from a cell, such as cell supernatant, cell debris, cell walls, DNA extract, enzymes or enzyme preparation or any preparation derived from cells by chemical, physical and/or enzymatic treatment, which is essentially free of living cells. Preferred extracts are enzymatically-active extracts. The composition of the invention may comprise one or several host cells of the invention or extract thereof containing the protease of the invention, and optionally one or several additional cells.

In a particular embodiment, the composition consists or comprises a lyophilized culture medium of a recombinant microorganism expressing and/or excreting a protease of the invention. In a particular embodiment, the powder comprises the protease of the invention and a stabilizing/solubilizing amount of glycerol, sorbitol or dextrin, such as maltodextrine and/or cyclodextrine, starch, Arabic gum, glycol such as propanediol, and/or salt.

### Uses of the proteases

It is a further object of the invention to provide methods using a protease of the invention for degrading and/or recycling in aerobic or anaerobic conditions polyester and/or polyester containing material, as plastic products made of or containing polyesters and/or producing biodegradable plastic products containing polyester. The proteases of the invention are particularly useful for producing biodegradable plastic products containing PLA and/or for degrading PLA and a plastic product comprising PLA.

It is therefore an object of the invention to use a protease of the invention, or corresponding host cell or extract thereof containing such protease, or composition, for the enzymatic degradation of a polyester or a polyester containing material, such as PLA or a PLA containing material.

It is another object of the invention to provide a method for degrading a polyester or a plastic product containing at least one polyester, wherein the polyester or the plastic product is contacted with a protease or host cell or composition of the invention. Advantageously, polyester(s) and/or polyester(s) of the polyester containing material is (are) depolymerized up to monomers and/or oligomers. In an embodiment of the method of degradation, at least one polyester is degraded to yield repolymerizable monomers and/or oligomers, which are advantageously retrieved in order to be used. In a preferred embodiment of the method of degradation, at least PLA is degraded to yield repolymerizable monomers and/or oligomers of lactic acid (LA), which are advantageously retrieved in order to be used for instance to produce new polymers of PLA.

In an embodiment, polyester(s) and/or polyester(s) of the polyester containing material is (are) fully degraded.

In a particular embodiment, the polyester is selected from polylactic acid (PLA), poly(glycolic acid) (PGA), poly(lactic-co-glycolic acid) (PLGA), polytrimethylene terephthalate (PTT), polybutylene terephthalate (PBT), polyethylene isosorbide terephthalate (PEIT), polyethylene terephthalate (PET), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA) and blends/mixtures thereof, preferably polylactic acid.

In a particular embodiment, the plastic product comprises at least one polyester selected from polylactic acid (PLA), polytrimethylene terephthalate (PTT), polybutylene terephthalate (PBT), polyethylene isosorbide terephthalate (PEIT), polyethylene terephthalate (PET), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA) and blends/mixtures of these materials, preferably polylactic acid.

In a particular embodiment, PLA or a plastic product containing PLA is contacted with a protease or host cell of the invention and PLA is degraded to monomers and/or oligomers of lactic acid. In a preferred embodiment, monomers and/or oligomers of lactic acid are recovered for recycling, polymerizing PLA or methanisation for instance.

The invention also relates to a method of producing monomers and/or oligomers from a polyester or polyester containing material, comprising exposing a polyester or polyester containing material to a protease of the invention, or corresponding host cell or extract thereof, or composition, and optionally recovering monomers and/or oligomers. The method of the invention is particularly useful for producing monomers such as lactic acid from plastic product containing PLA.

The time required for degrading a polyester or polyester containing material may vary depending on the polyester or polyester containing material itself (i.e., nature and origin of the plastic product, its composition, shape etc.), the type and amount of protease used, as well as various process parameters (i.e., temperature, pH, additional agents, etc.). One skilled in the art may easily adapt the process parameters to the polyester containing material.

Advantageously, the degrading process is implemented at a temperature comprised between 10°C and 90°C, e.g.; between 20°C to 70°C, between 20°C and 90°C, between 30°C to 60°C, between 40°C and 80°C, between 60°C and 80°C, between 70°C and 80°C, preferably at 45°C or at 75°C. More generally, the temperature is maintained below an inactivating temperature, which corresponds to the temperature at which the protease is inactivated and/or the recombinant microorganism does no more synthesize the protease. Preferably, the temperature is maintained below the glass transition temperature (Tg) of the polyester in the polyester containing material. In this embodiment, the degrading process is implemented at a temperature comprised below 80°C, preferably below 70°C, more preferably below 60°C, more preferably below 50°C, even more preferably at 45°C. More particularly, the process is implemented in a continuous way, at a temperature at which the protease can be used several times and/or recycled.

Advantageously, the degrading process is implemented at a pH comprised between 5 and 11, preferably at a pH between 6 and 10, more preferably at a pH between 6.5 and 9, even more preferably at a pH between 7 and 8.

In a particular embodiment, the polyester or polyester containing material may be pretreated prior to be contacted with the protease, in order to physically change its structure, so as to increase the surface of contact between the polyester and the enzyme.

Optionally, monomers and/or oligomers resulting from the depolymerization may be recovered, sequentially or continuously. A single type of monomers and/or oligomers or several different types of monomers and/or oligomers may be recovered, depending on the starting polyester containing material.

The recovered monomers and/or oligomers may be further purified, using all suitable purifying methods and conditioned in a repolymerizable form. Examples of purifying methods include stripping process, separation by aqueous solution, steam selective condensation, filtration and concentration of the medium after the bioprocess, separation, distillation, vacuum evaporation, extraction, electrodialysis, adsorption, ion exchange, precipitation, crystallization, concentration and acid addition dehydration and precipitation, nanofiltration, acid catalyst treatment, semi continuous mode distillation or continuous mode distillation, solvent extraction, evaporative concentration, evaporative crystallization, liquid/liquid extraction, hydrogenation, azeotropic distillation process, adsorption, column chromatography, simple vacuum distillation and microfiltration, combined or not.

The repolymerizable monomers and/or oligomers may then be used for instance to synthesize polyesters. Advantageously, polyesters of same nature are repolymerized. However, it is possible to mix the recovered monomers and/or oligomers with other monomers and/or oligomers, in order for instance to synthesize new copolymers. Alternatively, the recovered monomers may be used as chemical intermediates in order to produce new chemical compounds of interest.

It is a further object of the invention to provide a polyester or polyester containing material in which a protease of the invention and/or a recombinant microorganism expressing and/or excreting said protease and/or extract thereof containing such protease, and/or a composition of the invention is/are included. In a particular embodiment, such polyester containing material may be a plastic compound, a masterbatch composition and/or a plastic product. In the context of the invention, a "masterbatch composition" refers to a concentrated mixture of selected ingredients (e.g., active agents, additives, etc.) that can be used for introducing said ingredients into plastic compound or product in order to impart desired properties thereto. Masterbatch compositions may be solid or liquid. Preferably, masterbatch compositions of the invention contain at least 10% by weight of active ingredients, more preferably of protease or composition of the invention.

It is thus a further object of the invention to provide a plastic compound containing a protease of the invention and/or a recombinant microorganism expressing and/or excreting said protease or extract thereof containing such protease and/or a composition of the invention and at least one polyester. In a particular embodiment, the polyester is polylactic acid (PLA), preferably poly(L-lactic acid) (PLLA), poly(D-lactic acid) (PDLA) or poly(DL-lactic acid) (PDLLA). In a particular embodiment, the plastic compound may contain an additional polymer, preferably selected from polyesters such as PBAT, PCL, PET; polyolefins such as polyethylene, polypropylene or natural polymers such as starch, cellulose or flour; and blends/mixtures thereof. More particularly, the plastic compound may contain additional polymers selected from PBAT, flour or starch. In another particular embodiment, the polyester is polycaprolactone (PCL).

It is thus a further object of the invention to provide a masterbatch composition containing a protease of the invention and/or a recombinant microorganism expressing and/or excreting said protease or extract thereof containing such protease and/or a composition of the invention, and at least one polyester. In a particular embodiment, the polyester is polylactic acid (PLA), preferably poly(L-lactic acid) (PLLA), poly(D-lactic acid) (PDLA) or poly(DL-lactic acid) (PDLLA). In another particular embodiment, the polyester is preferably polycaprolactone (PCL).

In particular, the invention relates to a process for producing such polyester containing material (i.e., plastic compound, masterbatch composition or plastic product) comprising a step of mixing a polyester and a protease and/or recombinant microorganism of the invention or extract thereof containing such protease and/or a composition of the invention, able to degrade said polyester, at a temperature at which the polyester is in a partially or totally molten state so that the protease/ microorganisms are integrated into the very structure of the polyester containing material. In a particular embodiment, the process is an extrusion process.

For instance, the protease and/or the composition of the invention and the polyester may be mixed at a temperature between the glass transition temperature and the melting point of the polyester. Alternatively, the protease/ composition of the invention and the polyester may be mixed at a temperature corresponding to the melting point of said polyester, or above. In a particular embodiment, the protease/ composition and the polyester are mixed at a temperature between 40°C and 250°C, preferably between 50°C and 180°C. Alternatively, the polypeptide/ composition and the polyester are mixed at a temperature above 40°C, preferably above 50°C, even more preferably above 60°C.

In a preferred embodiment, the polyester is selected from polylactic acid (PLA), and the protease/ composition and PLA are mixed at a temperature between 60°C and 250°C, preferably between 100°C and 200°C, more preferably between 130°C and 180°C, even more preferably between 140°C and 160°C. Alternatively, the protease/ composition and PLA are mixed at a temperature above 80°C, preferably, above 100°C, even more preferably above 130°C, and below 180°C.

In another preferred embodiment, the polyester is selected from polycaprolactone (PCL), and the protease /composition and PCL are mixed at a temperature between 40°C and 100°C, preferably between 50°C and 80°C. Alternatively, the protease / composition and PCL are mixed at a temperature above 40°C, preferably, above 50°C, even more preferably above 55°C, and below 80°C.

More preferably, the mixing step is performed using extrusion, twin screw extrusion, single screw extrusion, injection-molding, casting, thermoforming, rotary molding, compression, calendering, ironing, coating, stratification, expansion, pultrusion, extrusion blow-molding, extrusion-swelling, compression-granulation, water-in-oil-in-water double emulsion evaporation, 3D printing or any techniques known by person skilled in the art.

The resulting plastic compound, masterbatch composition or plastic product integrates protease/microorganism or composition of the invention embedded in the mass of the compound, masterbatch composition or plastic product.

Advantageously, such plastic compound, masterbatch composition can be used for the production of polyester containing materials and/or plastic article that will thus include the polypeptide of the invention.

In a particular embodiment, the resulting plastic compound, masterbatch composition or plastic article is a biodegradable plastic compound, masterbatch composition or plastic article complying with at least one of the relevant standards and/or labels known by the person skilled in the art, such as standard EN 13432, standard ASTM D6400, OK Biodegradation Soil (Label Vingotte), OK Biodegradation Water (Label Vingotte), OK Compost (Label Vingotte), OK Home Compost (Label Vingotte).

Advantageously, the degrading process of the polyester containing material (i.e., plastic compound, masterbatch composition or plastic product) is implemented at a temperature comprised between 10°C and 50°C, preferably between 15°C and 40°C, more preferably between 20°C and 30°C, more preferably at 28°C, +/- 2°C.

Alternatively, the degrading process of the polyester containing material (i.e., plastic compound, masterbatch composition or plastic product) is implemented at a temperature comprised between 50°C and 60°C, more preferably at 55°C, +/- 2°C.

Classically, a protease of the invention may be used in detergent, food, animal feed and pharmaceutical applications.

More particularly, a protease of the invention may be used as a component of a detergent composition.

Detergent compositions include, without limitation, hand or machine laundry detergent compositions, such as laundry additive composition suitable for pre-treatment of stained fabrics and rinse added fabric softener composition, detergent composition for use in general household hard surface cleaning operations, detergent compositions for hand or machine dishwashing operations.

In a particular embodiment, a protease of the invention may be used as a detergent additive. The invention thus provides detergent compositions comprising a protease of the invention.

The present invention is also directed to methods for using a protease of the invention in animal feed, as well as to feed compositions and feed additives comprising a protease of the invention. The terms "feed" and "feed composition" refer to any compound, preparation, mixture, or composition suitable for, or intended for intake by an animal. In another particular embodiment, the protease of the invention is used to hydrolyze proteins, and to produce hydrolysates comprising peptides. Such hydrolysates may be used as feed composition or feed additives.

The invention also relates to a method of surface hydrolysis or surface functionalization of a polyester containing material, comprising exposing a polyester containing material to a protease of the invention, or corresponding recombinant cell or extract thereof, or composition. The method of the invention is particularly useful for increasing hydrophilicity, or water absorbency, of a polyester material. Such increased hydrophilicity may have particular interest in textiles production, electronics and biomedical applications.

### EXAMPLES

### Example 1 -Construction, expression and purification of proteases

### 1.1 Construction

The gene encoding for the non-matured parent protease (SEQ ID N°3) is cloned in the plasmid pET26b+ (EMD Millipore, Billerica, Massachusetts, USA), in frame with sequences encoding PelB signal peptide (MKYLLPTAAAGLLLLAAQPAMA - SEQ ID NO°4) upstream of the gene and a 6xhistidine tag (LEHHHHHH - SEQ ID N°5) downstream of the gene. E. coli One Shot^{®} BL21 DE3 (Life technologies, Carlsbad, California, USA) is transformed with the constructed plasmid. The obtained strain expresses the parent protease with a PelB leader sequence at the N-terminal and a 6X histidine Tag at the C-terminal of the protein. QuikChange II Site-Directed Mutagenesis kit is used according to the recommendations of the supplier to construct the variants (Santa Clara, California, USA).

### 1.2 Expression and purification of the proteases

Recombinant expression of the strains expressing the parent protease and variants thereof is realized in 100 mL TB medium at 37°C during 4 hours then at 21°C after IPTG addition at final concentration of 1 mM (Tartoff K. D. and Hobbs C. A., 1987, Improved Media for Growing Plasmid and Cosmid Clones, Bethesda Res. Lab. Focus, 9:12.). Cultures are stopped by centrifugation (8000 rpm, 20 minutes at 10°C) in an Avanti J-26 XP centrifuge (Beckman Coulter, Brea, USA). Cells are frozen at -80 °C during at least 2.5 hours and then suspended in 25 mL of Tris HCl buffer (Tris 0.1 M, pH 9). Lysonase^{™} Bioprocessing Reagent (EMD Millipore) can be used to lysate the cells, according to supplier's recommendation or sonication of the cell suspension during 2 minutes with 30% of amplitude (15s ON and 45s OFF cycles) using FB 705 sonicator (Fisherbrand, Illkirch, France). Cell suspension is centrifuged during 30 minutes at 11000 rpm and at 10°C. The soluble fraction is collected and submitted to cobalt affinity chromatography using Talon^{®} Metal Affinity resin (Clontech, CA, USA). Protein is eluted with 100mM imidazole in 20mM Tris-HCl, 300mM NaCl, pH 8.0. Imidazole is removed from purified extracts after a dialysis step or a desalting step with PD10 column (Sigma-Aldrich, GE17-0851-01) against Tris HCl buffer supplemented or not with calcium chloride (Tris 0.1 M, 0 or 5 mM CaCl2, pH 7.5 or pH 9 regulated at 45°C). The quality of the purification is assessed on SDS-PAGE by adding PMSF in excess to the protease solution, or after TCA precipitation, the expected protein size being around 29kDa. The level of expression of the purified protein is quantified using Bio-Rad Bradford protein assay according to manufacturer instructions (Lifescience Bio-Rad, France) and stored at +4°C.

### Example 2 - Evaluation of the degrading activity of the proteases

The specific degrading activities of parent protease and variants thereof is determined during PLA hydrolysis. 50 mg of a 500µm PLA powder (PLLA 001 - Natureplast) are weighed and introduced in dialysis tubing. The variants have the amino acid sequence as set forth in SEQ ID N°1, except specific substitutions, as listed. The enzymatic sample (1.5 mL of protease preparation containing a fixed concentration of enzyme of 2.5, 5, 10 or 15 mg/L in order to measure the accurate specific activity) is then added in the dialysis tubing before closing it and this latter is introduced in a glass bottle containing 25 mL of 0.1 M Tris-HCl buffer pH 9 or pH 7.5 (regulated at 45°C). The parent protease (SEQ ID N°1 and 6xhistidine tag) is used as a control.

The depolymerization started by incubating each sample at 45°C and 170 rpm in a Max Q 4450 incubator (Thermo Fisher Scientific, Inc. Waltham, MA, USA).

Initial rate of the depolymerization reaction in g of lactic acid and/or dimers of lactic acid generated / g of enzyme / hour is determined by samplings performed at different times during the first 24 hours and analyzed by Ultra High Performance Liquid Chromatography (UHPLC). To simplify the analysis, 3µl of NaOH 13M can be added to 200µl samples to ensure complete hydrolysis of dimers into lactic acid after 15 minutes at 95°C. If necessary, samples are diluted in 0.1 M Tris-HCl buffer pH 9 or pH 7.5 (regulated at 45°C). After filtration on 0.45 µm syringe filter, samples are loaded on UHPLC to monitor the liberation of lactic acid and dimers of lactic acid. Chromatography system used is an Ultimate 3000 UHPLC system (Thermo Fisher Scientific, Inc. Waltham, MA, USA) including a pump module, an autosampler, a column oven thermostated at 50°C, and a UV detector at 210 nm. The column used is an Aminex HPX-87H (300 mm × 7.8 mm), equipped with precolumn, (Supelco, Bellefonte, USA). Lactic acid and dimers of lactic acid are separated using a mobile phase H₂SO₄ 5 mM, at a flow rate of 0.5 mL.min⁻¹. Injection is 20 µL of sample. Lactic acid and dimers of lactic acid are measured according to standard curves prepared from commercial lactic acid (Sigma-Aldrich L1750-10G) and in house synthetized dimers of lactic acid in the same conditions than samples. The specific degrading activity of PLA hydrolysis (g of equivalent lactic acid, i.e., g of lactic acid and of dimer of lactic acid/hour/g of enzyme) is determined in the linear part of the hydrolysis curve.

Specific degrading activity of protease variants of the invention are shown in Table 1 below. The specific degrading activity of the protease of SEQ ID N°1 is used as a reference and considered as 100% specific degrading activity. The specific degrading activity is measured at 45°C and pH 9.

**Table 1: Specific degrading activity of variants of the invention as compared to parent protease**

| Variants | Specific degrading activity |
|---|---|
| V1 : replacement of amino acids [L210 to G213] with the sequence YTSSTA | 104% |
| V2 : replacement of amino acids [L210 to G213] with the sequence YTSETA | 117% |
| V3 : V2 + T17E | 110% |
| V4 : V2 + S194E + H197D | 95% |

### Example 3 - Evaluation of the thermostability of protease of the invention

The thermostability of proteases of the invention has been determined and compared to the thermostability of the protease of SEQ ID N°1.

Different methodologies have been used to estimate thermostability:
(1) Circular dichroism of proteins in solution;
(2) Differential Scanning Fluorimetry (DSF);
(3) Nano differential scanning fluorimetry (Nano-DSF)
(4) Residual polyester's degrading activity after protein incubation in given conditions of temperatures, times and buffers.

### 3.1 Circular Dichroism (CD)

Circular dichroism of proteins in solution is used to estimate thermostability.

Circular dichroism (CD) is performed on a J-815 CD spectrometer (JASCO) to determine and compare the melting temperature (*Tₘ*) of the protease of SEQ ID N°1 and protease variants of the invention. The *Tₘ* corresponds to the temperature at which 50% of the protein is denatured.

Protein sample was prepared at 0.2 mg/mL in buffer containing 100 mM Tris-HCl pH7.5 or pH9 supplemented or not with calcium chloride (0 - 5 - 10 mM). Experiments were performed in 1 mm optical path quartz cuvette (Starna Scientific Ltd, UK) and far-UV (195-260) CD spectra were first measured to determine two maxima intensities of CD corresponding to the correct folding of the protein.

Thermal denaturation curves of the proteins are obtained by monitoring the change in CD values at 220 nm as the temperature is increased. The rate of temperature increase is 1.5°C.min-1. The temperature of the midpoint of the transition, Tₘ, is calculated by curve fitting of the resultant CD values versus temperature data on the basis of a least-squares analysis using Sigmaplot version 11.0 software.

The *Tₘ* obtained reflects the thermostability of the given protein. The higher the *Tₘ* is, the more stable the variant is at high temperature.

The Tm of the parent protease of SEQ ID N°1 was evaluated at 53.4°C +/- 0.30°C without addition of CaCl₂.

### 3.2 Differential scanning fluorimetry (DSF)

Another method to estimate thermostability is differential scanning fluorimetry (DSF).

DSF was used to evaluate the thermostability of the parent protein (SEQ ID N°1) and variants thereof by determining their melting temperature (*Tₘ* ), temperature at which half of the protein population is unfolded. Protein samples were prepared at a concentration between 6 and 15 µM and stored in buffer A consisting of 0.1 M Tris-HCl pH 7.5 or 9.0 (complemented or not with calcium, concentration range between 0 and 20 mM), for these essays 5 to 10 mM of freshly prepared PMSF (0.2 M in EtOH) is added into the solution of protein. The SYPRO orange dye 5000 X stock solution in DMSO was first diluted to 500 X in water. Protein samples were loaded onto a white clear 96-well PCR plate (Bio-Rad cat# HSP9601) with each well containing a final volume of 25 µl. The final concentration of protein, PMSF and SYPRO Orange dye in each well were 5 to 10 µM (0.15 to 0.30 mg/ml), 5 to 10 mM and 20 X respectively. Loaded volumes per well were as follow: 24 µL of protein in buffer A with PMSF and 1 µL of the 500 X Sypro Orange diluted solution. The PCR plates were then sealed with optical quality sealing tape and spun at 1 000 rpm for 1 min at room temperature. DSF experiments were then carried out using a CFX96 real-time PCR system set to use the 450/490 nm excitation and 560/580 nm emission filters. The samples were heated from 25 to 100°C at the rate of 0.3°C/second. A single fluorescence measurement was taken every 0.03 second. Melting temperatures were determined from the peak(s) of the first derivatives of the melting curve using the Bio-Rad CFX Manager software. The Tm of the parent protease of SEQ ID N°1 was evaluated at 53.8°C using DSF.

Thermostabilities of protease variants of the invention are shown in Table 2 below, expressed in Tm values and evaluated according to Example 3.2 (at pH 7.5 and with PMSF). The gain of Tm as compared to the protease of SEQ ID N°1 is indicated in brackets.

**Table 2: Tm of the proteases of the invention compared to SEQ ID N°1 (measured with DSF)**

| Variants | Tm |
|---|---|
| V2 : replacement of amino acids [L210 to G213] with the sequence YTSETA | 55.0°C (+1.2°C) |
| V3 : V2 + T17E | 56.0°C (+2.2°C) |
| V4 : V2 + S194E + H197D | 56.8°C (+3.0°C) |
| V5 : S194E + H197D + S264P | 58.40°C (+4.6°C) |
| V6 : V2 + T17E + S194E + H197D | 56.9°C (+3.1°C) |

### 3.3 Nano differential scanning fluorimetry (Nano-DSF)

The nanoDSF uses the intrinsic fluorescence of protein (tryptophan or tyrosin) to monitor they unfolding and obtain a *Tₘ* value. Protein samples were prepared at a concentration between 8 and 10 µM and stored in buffer A consisting of 0.1 M Tris-HCl pH 7.5 or 9.0 (complemented or not with calcium chloride 0 to 10 mM). 10 µL of protein solutions were loaded onto a dedicated capillary sealed before loading to the instrument. NanoDSF experiments were then carried out using a Prometheus NT.Plex system (NanoTemper Technologies, Germany) set for detection of the 330 and 350 nm fluorescence. The samples were heated from 15 to 110°C at the rate of 1°C/min and laser power was 20 or 50%. *Tₘ* were determined from the peak(s) of the derivative of the ratio of fluorescence at 330/350 nm using the Prometheus software.

The Tm value of the parent protease of SEQ ID N°1 was evaluated at 58.9°C without calcium chloride, 74.9°C with 5 mM of calcium chloride (evaluated according to Example 3.3 at pH 9).

Thermostabilities of protease variants of the invention are shown in Table 3 below, expressed in Tm values and evaluated according to Example 3.3 at pH 9. The gain of Tm as compared to the protease of SEQ ID N°1 is indicated in brackets.

**Table 3: Tm of the proteases of the invention compared to SEQ ID N°1 (measured with Nano-DSF at pH 9).**

| Variants | Without CaCl2 | With 5 mm CaCl2 |
|---|---|---|
| V2 : replacement of amino acids [L210 to G213] with the sequence YTSETA | 60.2°C (+1.3°C) | 76.4°C (+1.5°C) |
| V6 : V2 + T17E + S194E + H197D | 61.6°C (+2.7°C) | 77.3°C (+2.4°C) |

### 3.4 Residual polyester's degrading activity after protein incubation in given conditions of temperature and times

Thermal stabilities of parent protease and variants were determined by measurement of the residual specific degrading activity (PLA hydrolysis as described in Example 2) recovered after a heat shock. The heat shocks were performed as follow: an enzymatic sample containing a fixed enzyme concentration (0.1 or 0.15 g/L) in 0.1 M Tris-HCl buffer pH 7.5 (regulated at 45°C), with or without addition of calcium chloride up to 20 mM, was immersed in a water-bath adjusted at 70°C during a given time (30 or 60 minutes). The samples were immediately placed on ice after the heat shock. After a step of dilution of the enzyme (up to 0.05 g/L), the specific degrading activities (PLA hydrolysis) recovered after the heat shocked and non-heat shocked samples were measured as detailed in Example 2.

Thermal stabilities of variants as compared to the protease of SEQ ID N°1 can be validated if the residual specific degrading activity of protease variants after the heat shock is higher than the residual specific degrading activity of parent protease after the heat shock.

Residual specific degrading activity of protease variants of the invention after the heat shock are shown in Table 4 below. The residual specific degrading activity of the protease of SEQ ID N°1 after the heat shock is used as a reference and considered as 100% specific degrading activity.

**Table 4: Residual specific activity of the proteases of the invention after heat shock at 70°C compared to SEQ ID N°1**

| Variant | Residual specific degrading activity after heat shock |
|---|---|
| V1 : replacement of amino acids [L210 to G213] with the sequence YTSSTA | 247% |
| V2 = replacement of amino acids [L210 to G213] with the sequence YTSETA | 500% |
| V3 : V2 + T17E | 1417% |
| V4 : V2 + S194E + H197D | 392% |
| V5 : S194E + H197D + S264P | 209% |
| V6 : V2 + T17E + S194E + H197D | 650% |
| V7 : N253C + N272C | 942% |

Thermal stabilities of variants as compared to the protease of SEQ ID N°1 can also be validated if the percentage of residual specific degrading activity of protease variants after the heat shock (defined as the ratio between the specific activity of the enzyme before the heatshock and after the heatshock) is higher than the percentage of residual specific degrading activity of parent protease after the heat shock.

Improvement factors of said specific degrading activity after heat shock are shown in Table 5 below. The improvement factors of the residual degrading activities are expressed as a ratio between the percentage of residual activity of the variant and the percentage of residual activity of the parent protein (SEQ ID N°1).

**Table 5: Improvement factor of the residual activity of the proteases of the invention after heat shock at 70°C compared to SEQ ID N°1**

| Variant | Improvement factor of the residual activity |
|---|---|
| V1 : replacement of amino acids [L210 to G213] with the sequence YTSSTA | 2.29 |
| V2 : replacement of amino acids [L210 to G213] with the sequence YTSETA | 4.12 |
| V3 : V2 + T17E | 12.62 |
| V4 : V2 + S194E + H197D | 4.04 |
| V5 : S194E + H197D + S264P | 3.92 |
| V6: V2 + T17E + S194E + H197D | 7.62 |
| V7 : N253C + N272C | 37.51 |
| V8 : N262C + T266C | 3.73 |
| V9 : A169C + T176C | 7.30 |
| V10 : W9C + S182C | 1.16 |
| V11 : N173C + F243C | 7.96 |
| V12 : A187C + S203C | 4.83 |
| V13 : C164A + A172C | 4.22 |

## Claims

1. A protease which comprises an amino acid sequence which (i) has at least 90%, 95%, 96%, 97%, 98% or 99% identity to the full length amino acid sequence set forth in SEQ ID N°1, and (ii) has at least one combination of substitutions selected from the group consisting of N262C + T266C, A169C + T176C, W9C + S182C, N173C + F243C, A187C + S203C, C164A + A172C, S194E + H197D + S264P and N253C + N272C, or comprises at least the replacement of the portion of amino acid sequence corresponding to residues [L210 to G213] with the sequence YTSETA or YTSSTA , (iii) comprises the combinations D40 + H71 + S221 as in the parent protease, wherein the positions are numbered by reference to the amino acid sequence set forth in SEQ ID N°1, and (iv) has a polyester degrading activity, and (v) exhibits increased thermostability compared to the protease of SEQ ID N°1.

2. The protease according to claim 1, wherein said protease further comprises at least one amino acid residue selected from C68, C100, C164 or C195 forming disulfide bonds, as in the parent protease.

3. The protease according to claim 1 or 2, wherein said protease comprises at least one combination selected from D40 + H71 + S221 + C68 + C100, D40 + H71 + S221 + C164 + C195 or D40 + H71 + S221 + C68 + C100 + C164 + C195 as in the parent protease.

4. The protease according to any one of claims 1 to 3, wherein said protease comprises the replacement of the portion of amino acid sequence corresponding to residues [L210 to G213] with the sequence YTSETA or YTSSTA and further comprises at least a substitution or combination of substitutions selected from T17E, S194E + H197D, and T17E + S194E + H197D.

5. The protease according to any one of the previous claims, wherein said protease exhibits an increased thermostability compared to the protease of SEQ ID N°1 at temperatures between 40°C to 90°C, preferably at 70°C, +/- 5°C.

6. A method of degrading a plastic product containing at least one polyester comprising
(a) contacting the plastic product with a protease according to any one of claims 1 to 5 or an host cell comprising and expressing a nucleic acid encoding the protease according to any one of claims 1 to 5 or a composition comprising the protease according to any one of claims 1 to 5; and, optionally
(b) recovering monomers and/or oligomers.

7. The method of claim 6, wherein the polyester is preferably selected from the group consisting in polylactic acid (PLA), polytrimethylene terephthalate (PTT), polybutylene terephthalate (PBT), polyethylene isosorbide terephthalate (PEIT), polyethylene terephthalate (PET) polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA), poly(glycolic acid) (PGA), poly(lactic-co-glycolic acid) (PLGA) and blends/mixtures thereof, preferably polylactic acid.

8. A plastic compound containing (i) at least one polyester, preferably at least polylactic acid and (ii) a protease of any one of claims 1 to 5 or a host cell comprising and expressing a nucleic acid encoding the protease according to any one of claims 1 to 5 or extract thereof comprising said protease or a composition comprising the protease according to any one of claims 1 to 5, wherein the protease is advantageously able to degrade at least one polyester of the plastic compound.

9. Use of a protease according to any one of claims 1 to 5 or host cell comprising and expressing a nucleic acid encoding the protease according to any one of claims 1 to 5 or a composition comprising the protease according to any one of claims 1 to 5, for degrading a polyester containing material, preferably for degrading a polylactic acid containing material.

## Patentansprüche

1. Protease, die eine Aminosäuresequenz umfasst, die (i) mindestens 90%, 95%, 96%, 97%, 98% oder 99% Identität mit der in SEQ ID Nr. 1 angegebenen Aminosäuresequenz voller Länge aufweist und (ii) mindestens eine Kombination von Substitutionen aufweist, ausgewählt aus der Gruppe bestehend aus N262C + T266C, A169C + T176C, W9C + S182C, N173C + F243C, A187C + S203C, C164A + A172C, S194E + H197D + S264P und N253C + N272C, oder mindestens den Austausch des Teils der Aminosäuresequenz, der den Resten [L210 bis G213] entspricht, durch die Sequenz YTSETA oder YTSSTA umfasst, (iii) die Kombinationen D40 + H71 + S221 wie in der ursprünglichen Protease umfasst, wobei die Positionen anhand der Aminosäuresequenz in SEQ ID Nr. 1 nummeriert sind, und (iv) eine polyesterabbauende Aktivität aufweist und (v) eine erhöhte Thermostabilität im Vergleich zur Protease der SEQ ID Nr. 1 aufweist.

2. Die Protease nach Anspruch 1, wobei diese Protease zusätzlich mindestens einen Aminosäurerest ausgewählt aus C68, C100, C164 oder C195 enthält, der wie in der ursprünglichen Protease Disulfidbrücken bildet.

3. Die Protease nach Anspruch 1 oder 2, wobei diese Protease mindestens eine Kombination ausgewählt aus D40 + H71 + S221 + C68 + C100, D40 + H71 + S221 + C164 + C195 oder D40 + H71 + S221 + C68 + C100 + C164 + C195 wie in der Stammprotease umfasst.

4. Die Protease nach einem der Ansprüche 1 bis 3, wobei diese Protease den Austausch des Teils der Aminosäuresequenz, der den Resten [L210 bis G213] entspricht, durch die Sequenz YTSETA oder YTSSTA umfasst und zudem mindestens eine Substitution oder Kombination von Substitutionen ausgewählt aus T17E, S194E + H197D und T17E + S194E + H197D aufweist.

5. Die Protease nach einem der vorhergehenden Ansprüche, wobei diese Protease im Vergleich zur Protease der SEQ ID Nr. 1 eine erhöhte Thermostabilität bei Temperaturen zwischen 40 °C und 90 °C, vorzugsweise bei 70 °C, ± 5 °C, aufweist.

6. Verfahren zum Abbau eines Kunststoffprodukts, das mindestens einen Polyester enthält, umfassend
(a) Inkontaktbringen des Kunststoffprodukts mit einer Protease nach einem der Ansprüche 1 bis 5 oder einer Wirtszelle, die eine für die Protease nach einem der Ansprüche 1 bis 5 kodierende Nukleinsäure umfasst und exprimiert, oder eine Zusammensetzung, die die Protease nach einem der Ansprüche 1 bis 5 umfasst; und optional
(b) Gewinnen von Monomeren und/oder Oligomeren.

7. Verfahren nach Anspruch 6, wobei der Polyester vorzugsweise aus der Gruppe ausgewählt wird, die aus Polymilchsäure (PLA), Polytrimethylenterephthalat (PTT), Polybutylenterephthalat (PBT), Polyethylenisosorbidterephthalat (PEIT), Polyethylenterephthalat (PET), Polyhydroxyalkanoat (PHA), Polybutylensuccinat (PBS), Polybutylensuccinatadipat (PBSA), Polybutylenadipatterephthalat (PBAT), Polyethylenfuranoat (PEF), Polycaprolacton (PCL), Polyethylenadipat (PEA), Polyglykolsäure (PGA), Polymilchsäure-co-glykolsäure (PLGA) und Mischungen/Mixturen davon besteht, vorzugsweise Polymilchsäure.

8. Kunststoffverbindung, enthaltend (i) mindestens einen Polyester, vorzugsweise mindestens Polymilchsäure, und (ii) eine Protease nach einem der Ansprüche 1 bis 5 oder eine Wirtszelle, die eine für die Protease nach einem der Ansprüche 1 bis 5 kodierende Nukleinsäure umfasst und exprimiert, oder einen diese Protease enthaltenden Extrakt davon oder eine die Protease nach einem der Ansprüche 1 bis 5 enthaltende Zusammensetzung, wobei die Protease vorteilhafterweise mindestens einen Polyester der Kunststoffverbindung abbauen kann.

9. Verwendung einer Protease nach einem der Ansprüche 1 bis 5 oder einer Wirtszelle, die eine für die Protease nach einem der Ansprüche 1 bis 5 kodierende Nukleinsäure umfasst und exprimiert, oder eine Zusammensetzung, die die Protease nach einem der Ansprüche 1 bis 5 umfasst, zum Abbau eines polyesterhaltigen Materials, vorzugsweise zum Abbau eines polymilchsäurehaltigen Materials.

## Revendications

1. Protéase qui comprend une séquence d'acides aminés qui (i) a une identité d'au moins 90 %, 95 %, 96 %, 97 %, 98 % ou 99 % avec la séquence d'acides aminés de longueur entière exposée dans la SEQ ID N°1, et (ii) au moins une combinaison de substitutions choisie dans le groupe consistant en N262C + T266C, A169C + T176C, W9C + S182C, N173C + F243C, A187C + S203C, C164A + A172C, S194E + H197D + S264P et N253C + N272C, ou comprend au moins le remplacement de la partie de la séquence d'acides aminés correspondant aux résidus [L210 à G123] par la séquence YTSETA ou YTSSTA, (iii) comprend les combinaisons D40 + H71 + S221 comme dans la protéase parente, où les positions sont numérotées par référence à la séquence d'acides aminés exposée dans la SEQ ID N°1, et (iv) présente une activité de dégradation du polyester, et (v) présente une thermostabilité accrue par rapport à la protéase de SEQ ID N°1.

2. Protéase selon la revendication 1, où ladite protéase comprend en outre au moins un résidu d'acide aminé choisi parmi C68, C100, C164 ou C195 formant des liaisons disulfure, comme dans la protéase parente.

3. Protéase selon la revendication 1 ou 2, où ladite protéase comprend au moins une combinaison choisie parmi D40 + H71 + S221 + C68 + C100, D40 + H71 + S221 + C164 + C195 ou D40 + H71 + S221 + C68 + C100 + C164 + C195 comme dans la protéase parente.

4. Protéase selon l'une quelconque des revendications 1 à 3, où ladite protéase comprend le remplacement de la partie de la séquence d'acides aminés correspondant aux résidus [L210 à G123] par la séquence YTSETA ou YTSSTA et comprend en outre au moins une substitution ou une combinaison de substitutions choisie parmi T17E, S194E + H197D, et T17E + S194E + H197D.

5. Protéase selon l'une quelconque des revendications précédentes, où ladite protéase présente une thermostabilité accrue par rapport à la protéase de SEQ ID N°1 à des températures comprises entre 40°C à 90°C, de préférence à 70°C, +/- 5°C.

6. Méthode de dégradation d'un produit plastique contenant au moins un polyester comprenant
(a) la mise en contact du produit plastique avec une protéase selon l'une quelconque des revendications 1 à 5 ou une cellule hôte comprenant et exprimant un acide nucléique codant pour la protéase selon l'une quelconque des revendications 1 à 5 ou une composition comprenant la protéase selon l'une quelconque des revendications 1 à 5 ; et, éventuellement
(b) la récupération des monomères et/ou oligomères.

7. Méthode selon la revendication 6, dans laquelle le polyester est de préférence choisi dans le groupe consistant en l'acide polylactique (PLA), le polytriméthylène téréphtalate (PTT), le polybutylène téréphtalate (PBT), le polyéthylène isosorbide téréphtalate (PEIT), le polyéthylène téréphtalate (PET), le polyhydroxyalcanoate (PHA), le polybutylène succinate (PBS), le polybutylène succinate adipate (PBSA), le polybutylène adipate téréphtalate (PBAT), le polyéthylène furanoate (PEF), la polycaprolactone (PCL), le polyéthylène adipate (PEA), l'acide polyglycolique (PGA), l'acide polylactique-co-glycolique (PLGA) et des mélanges/mix de ceux-ci, de préférence l'acide polylactique.

8. Composé plastique contenant (i) au moins un polyester, de préférence au moins l'acide polylactique et (ii) une protéase selon l'une quelconque des revendications 1 à 5 ou une cellule hôte comprenant et exprimant un acide nucléique codant pour la protéase selon l'une quelconque des revendications 1 à 5 ou un extrait de celle-ci comprenant ladite protéase ou une composition comprenant la protéase selon l'une quelconque des revendications 1 à 5, où la protéase est avantageusement apte à dégrader au moins un polyester du composé plastique.

9. Utilisation d'une protéase selon l'une quelconque des revendications 1 à 5 ou cellule hôte comprenant et exprimant un acide nucléique codant pour la protéase selon l'une quelconque des revendications 1 à 5 ou une composition comprenant la protéase selon l'une quelconque des revendications 1 à 5, pour la dégradation d'un matériau contenant du polyester, de préférence pour la dégradation d'un matériau contenant de l'acide polylactique.
